# FASCICULE DE BREVET EUROPEEN

(11) **EP 0 408 739 B1**
(45) Date de publication et mention de la délivrance du brevet: **31.08.1994**
(21) Numéro de dépôt: 90903598.2
(22) Date de dépôt: 08.02.1990
(51) Int. Cl.: A61F 5/02, A61B 17/58

(54) **DISPOSITIF D'ETAIEMENT DU RACHIS**
STÜTZVORRICHTUNG FÜR DIE WIRBELSÄULE
A SUPPORTING DEVICE FOR THE SPINAL COLUMN

(30) Priorité: 09.02.1989 FR 8901923
(43) Date de publication de la demande: 23.01.1991
(73) Titulaire: STRYKER CORPORATION (a Michigan corporation), Kalamazoo, Michigan 49002 (US); MISSENARD, Gilles, F-75016 Paris (FR); LAPRESLE, Philippe, F-92200 Neuilly-sur-Seine (FR)
(72) Inventeur: VIGNAUD, Jean-Louis, F-33400 Talence (FR); SACRISTE, Jean-François, F-33115 Le Pyla-sur-Mer (FR); MISSENARD, Gilles, F-75016 Paris (FR); LAPRESLE, Philippe, F-92200 Neuilly-sur-Seine (FR)
(74) Mandataire: Martin, Jean-Jacques
(86) Numéro de dépôt international: FR9000096
(87) Numéro de publication internationale: WO9009156

(56) Documents cités:
- EP-A- 0 128 058
- EP-A- 0 283 373
- EP-A- 0 348 272
- FR-A- 2 506 605
- FR-A- 2 559 378
- FR-A- 2 624 720
- GB-A- 2 173 104
- US-A- 4 743 260

## Description

La présente invention concerne un dispositif permettant, par implantation d'une instrumentation rachidienne par vis diapason, de contenir, réduire, s'adapter et rétablir les courbures physiologiques du rachis, au moyen de plusieurs vis dont la tp̂te en forme de U permet de recevoir un élément de de solidarisation des différentes vis entre elles.

Habituellement, on implantait des vis pédiculaires réunies par des plaques. De ce fait, on était dépendant de l'écartement des trous des plaques. D'où une bonne cotention dis une faible réduction.

On implantait aussi des crochets posés sur les arcs postérieurs évoluants sur des tiges avec des systèmes de blocage complexes. Des systèmes permettent une bonne réduction et une bonne contention mais exigent d'une part le respect des arcs postérieurs vertébraux, et d'autre part des manoeuvres délicates de va et vient dangereuses pour le patient et peu pratiques pour l'opérateur (voir 5th proceeding of the international congress on Cortel-Dubousset p.239 fig. 17 et le document GB-A-2173104

Le dispositif selon l'invention permet de remédier à tous ces inconvénients.

Il comporte en effet plusieurs vis composés de trois segments ; pointe-corps-tête particuliers, d'un boulon, d'un capuchon, et d'un élément de solidarisation homolatéral.

La structure et la conformation de ce matériel a été spécialement crée à cet effet.

La vis est construite dans des matériaux homologués pour la chirurgie afin d'éviter les ruptures du matériel au niveau de sa pénétration dans le corps vertébral.

La pointe de la vis est de forme carrée pyramidale renversée. Le corps de la vis de forme cônique est porteur d'un filetage de type cortical qui permet un meilleur blocage de l'implant dans la région la plus fragile du pédicule vertébral.

De plus, cette conicité renforce la solidité du corps de la vis au niveau de la tête de vis.

La tête de la vis a une forme de diapason en U pour recevoir l'élément de solidarisation des diffférentes vis entre elles. Le fond du U est légèrement arrondi pour permettre de fixer l'élément de solidarisation dans plusieurs positions ; ce qui facilite aussi le blocage de l'élément de solidarisation.

Le U est fileté au niveau de la partie interne des branches pour permettre le vissage in situ d'un boulon vérouillant l'élément de solidarisation. Le risque de ce système étant l'écartement des branches du U lors du vissage, on y remédie par la mise en place d'un capuchon venant parfaitement s'adapter sur la partie extérieure lisse des branches du U. Ce capuchon comporte un orifice circulaire pour permettre le serrage et le blocage définitif du boulon bloquant la tige de solidarisation.

Le boulon se caractérise par une forme cylindrique filetée portant à son extrémité une pointe cônique mâle venant s'adapter parfaitement dans les crans coniques femelles de la tige de solidarisation. La partie opposée du boulon comportant une cavité de forme hexagonale lui permettant de recevoir une clef de serrage.

L'élement de solidarisation est dans un matériau homologué pour l'implantation chirurgicale. C'est une tige de section cylindrique d'un diamètre suffisant pour résister aux contraintes et efforts du rachis, crantée pour permettre un réglage micrométrique des différentes vis entre elles. Chaque cran est de forme cônique femelle pour recevoir la pointe du boulon Suivant le réglage à obtenir le crantage sera d'un pas variable.

Le diamètre de l'élément de solidarisation suffit à le rendre résistant tout en permettant un modelage aisé pour s'adapter ou rétablir les courbures physiologiques du rachis. De plus, sa section circulaire permet la rotation avant son verrouillage celui-ci se faisant après un positionnement correct de l'élément de solidarisation en serrant le boulon des différentes vis homolatérales.

Cette tige est de mise en place facile du fait de la conformation des têtes de vis car elle peut être positionnée directement au fond du U sans mouvement forcé ni nécessité de coulissage par va et vient.

La structure même de l'implant diapason permet en modifiant la taille de la vis ou en adaptant la tête de vis à d'autres systèmes d'ostéosynthèse de l'arc postérieur de pratiquer des fixations vertébrales sur toute la hauteur du rachis quels que soient le niveau et le type de vertèbres instrumentées en conservant le même élément de solidarisation.

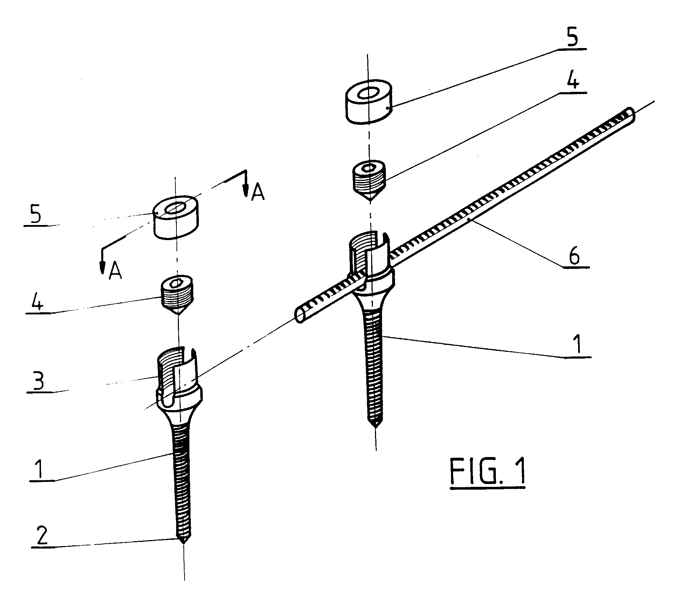

Le dispositif représenté sur la fig 1 comporte deux vis côniques (1) avec une pointe (2), un corps (1) un diapason en forme de U (3), un boulon (4), un capuchon (5), un élément de solidarisation (6).

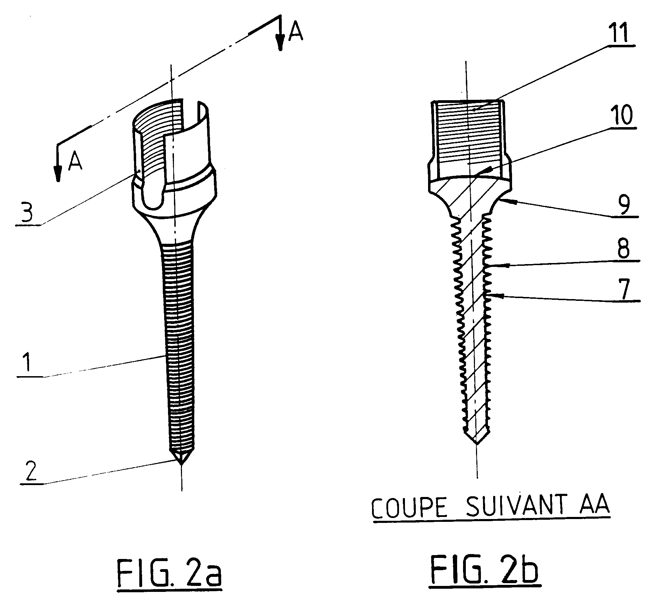

Le dispositif représenté sur les fig. 2a et 2b comporte une vis cônique de type cortical (1) avec une pointe carrée (2) à son extrémité inférieure.

Le corps de la vis (1) comporte une double conicité : à savoir, conicité de l'âme de la vis (7) différente de celle du sommet du filet (8).

La tête diapason en forme de U (3) est reliée au corps de la vis par un rayon (9) renforçant la solidité du corps de la vis au niveau de sa jonction avec le pédicule vertébral. Le fond du U est légèrement arrondi (10) pour permettre un blocage multi-axial de l'élément de solidarisation (6).

Le diamètre supérieur du U (3) est plus petit à sa partie supérieure qu'à la partie inférieure. Ceci permettant de recevoir par ajustement glissant le capuchon (5).

La partie intérieure du U est filetée (11) et reçoit un boulon (4) permettant le blocage de l'élément de solidarisation.

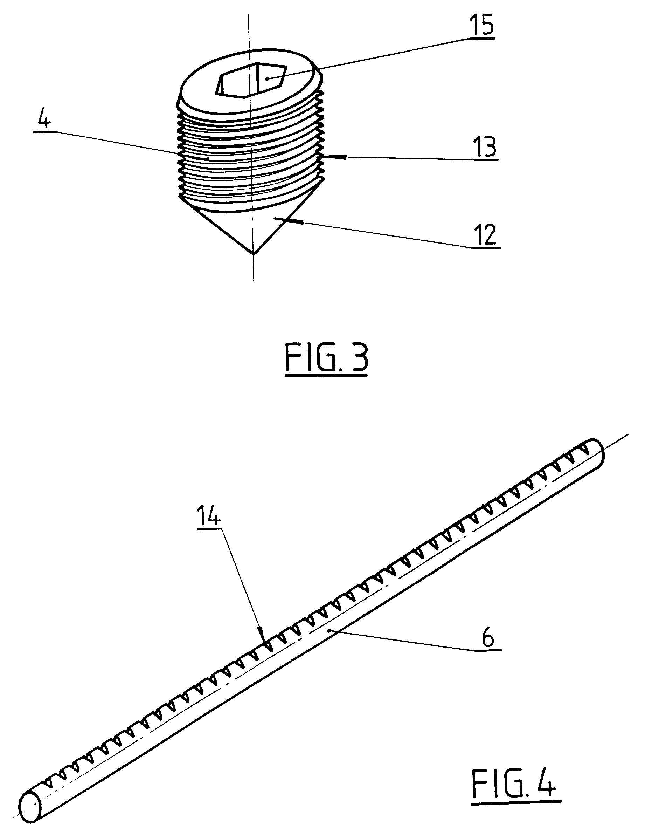

Le dispositif représenté sur la fig 3 comporte un boulon (4) avec à son extrémité inférieure une partie cônique (12) ; une partie cylindrique filetée (13) venant se visser dans le corps du U (3) en s'adaptant aux filets (11).

La partie cônique (12) vient s'adapter à la partie cônique femelle (14) de l'élément de solidarisation (6).

La partie supérieure du boulon (4) comporte une cavité hexagonale creuse (15) permettant de recevoir une clef de serrage.

Le dispositif représenté sur la fig.4 comporte un élément de solidarisation (6) des différentes vis des fig. 2-3-4.

Cet élément de solidarisation (6) est de section cylindrique.

Cet élément de solidarisation (6) est cranté (14) pour permettre le réglage micrométrique à l'aide du boulon (4) des différentes vis entre elles.

Les crans de forme cônique femelle (14) reçoivent la pointe du boulon (12).

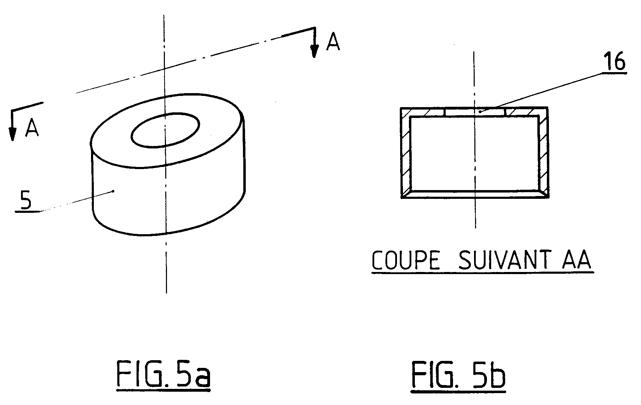

Le dispositif représenté sur les fig. 5a et 5b comporte un capuchon (5) cylindrique venant s'adapter par glissement serré sur le sommet du U (3).

La partie supérieure comporte un orifice (16) permettant le passage de la clef de serrage du boulon (4).

## Revendications

1. Dispositif d'étaiement du rachis comprenant plusieurs vis (1) présentant une tête en forme de U (3) à l'intérieur de laquelle se pose une tige de solidarisation (6) bloquée par un boulon (4) vissé dans un filetage réalisé dans la partie interne (11) des branches du U et muni d'une cavité (15) de réception d'une clé de serrage, caractérisé en ce que la partie supérieure de la tête reçoit un capuchon (5) adapté par ajustement glissant sur la partie extérieure lisse des branches du U et muni d'un orifice (16) permettant le passage de ladite clé de serrage dudit boulon (4).

2. Dispositif suivant la revendication 1, caractérisé en ce que ladite partie supérieure de la tête de vis (3) présente un diamètre plus petit que celui de la partie inférieure, ledit capuchon (5) venant s'adapter par ajustement glissant sur la partie de moindre diamètre de ladite vis.

3. Dispositif suivant la revendication 1 ou 2, caractérisé en ce que la tige de solidarisation (6) comporte des crans de forme conique femelle (14) situés sur la périphérie de la tige, usinés selon différents pas et dans lesquels vient se bloquer l'extrémité inférieure conique dudit boulon (4).

4. Dispositif suivant l'une des revendications 1 à 3, caractérisé en ce que la vis (1) comporte une conicité différente entre l'âme de la vis (7) et le sommet des filets (8).

5. Dispositif selon l'une des revendications 1 à 4, caractérisé en ce que le fond du U (3) est légèrement arrondi (10) dans les deux plans pour permettre un blocage multi-axial de ladite tige de solidarisation(6).

## Claims

1. A supporting device for the spinal column, the device comprising a plurality of screws (1) each having a U-shaped head (3) within which a joining rod (6) is placed and is held by a bolt (4), which is screwed into threads provided on the inside portions (11) of the branches of the U-shape and which is provided with a cavity (15) for receiving a chuck key, the device being characterized in that the top portion of the head receives a cap (5) installed as a sliding fit on the smooth outside portions of the branches of the U-shape and provided with an orifice (16) allowing said chuck key for said bolt (4) to pass through.

2. A device according to claim 1, characterized in that said top portion of the screw head (3) has a diameter smaller than that of its bottom portion, said cap (5) being installed as a sliding fit onto the smaller diameter portion of said screw.

3. A device according to claim 1 or 2, characterized in that the joining rod (6) includes female conical notches (14) situated on the periphery of the rod, which notches are machined at different pitches , and in which the conical bottom ends of said bolts (4) are held.

4. A device according to any one of claims 1 to 3, characterized in that each screw (1) exhibits different tapers for the core of the screw (7) and for the tops of its threads (8).

5. A device according to any one of claims 1 to 4, characterized in that the bottom of each U-shape (3) is slightly rounded (10) in both planes to allow for said joining rod (6) to be held multi-axially.

## Patentansprüche

1. Vorrichtung zum Abstutzen der Wirbelsäule, mit mehreren Schrauben (1), welche einen Kopf in Form eines U (3) haben, in dessen Innerem eine Verstärkungsstange (6) ruht, von einem Dornbolzen (4) verriegelt, der in ein Gewinde eingeschraubt ist, das in dem Innenteil (11) der Beine des U ausgebildet ist, und der mit einem Hohlraum (15) für einen Futterschlüssel versehen ist, dadurch gekennzeichnet, daß der obere Teil des Kopfes eine Kappe (5) aufnimmt, die durch gleitendes Aufziehen auf den äußeren glatten Teil der Beine des U gesetzt ist und mit einer Öffnung (16) versehen ist, die das Durchgreifen des Futterschlüssels für den Bolzen (4) erlaubt.

2. Vorrichtung nach Anspruch 1, dadurch gekennzeichnet, daß der obere Teil des Kopfes der Schraube (3) einen Durchmesser hat, der kleiner ist als der des unteren Teiles, wobei die Kappe (5) durch gleitendes Aufziehen auf dem Teil der Schraube mit geringerem Durchmesser in Anlage kommt.

3. Vorrichtung nach Anspruch 1 oder 2, dadurch gekennzeichnet, daß die Verstärkungsstange (6) aufnehmende Ausschnitte (14) konischer Form aufweist, die an der Umfangsfläche der Stange liegen, die nach unterschiedlichen Schritten gebildet sind und in die das konische untere Ende des Dornbolzens (4) zum Verriegeln kommt.

4. Vorrichtung nach einem der Ansprüche 1 bis 3, dadurch gekennzeichnet, daß die Schraube (1) eine unterschiedliche Konizität zwischen dem Inneren der Schraube (7) und dem höchsten Bereich der Gewinde (8) aufweist.

5. Vorrichtung nach einem der Ansprüche 1 bis 4, dadurch gekennzeichnet, daß die Sohle des U (3) in den beiden Ebenen leicht gerundet (10) ist, um eine multiaxiale Verriegelung der Verstärkungsstange (6) zu erlauben.
